# EUROPÄISCHE PATENTSCHRIFT

(11) **EP 0 612 736 B1**
(45) Veröffentlichungstag und Bekanntmachung des Hinweises auf die Patenterteilung: **14.04.1999**
(21) Anmeldenummer: 94102191.7
(22) Anmeldetag: 12.02.1994
(51) Int. Cl.: C07D 263/34

(54) **Verfahren zur Herstellung von Oxazolderivaten**
Process for the preparation of oxazole derivatives
Procédé de préparation de dérivés d'oxazole

(30) Priorität: 25.02.1993 CH 578/93
(43) Veröffentlichungstag der Anmeldung: 31.08.1994
(73) Patentinhaber: F. HOFFMANN-LA ROCHE AG, 4002 Basel (CH)
(72) Erfinder: Bonrath, Werner, D-79106 Freiburg (DE); Karge, Reinhard, D-79219 Staufen (DE); Pauling, Horst, CH-4103 Bottmingen (CH)
(74) Vertreter: Kellenberger, Marcus, Dr.

(56) Entgegenhaltungen:
- EP-A- 0 010 697
- EP-A- 0 492 233
- US-A- 3 222 374
- SYNTHESIS. Nr. 8 , 1980 , STUTTGART DE Seiten 657 - 658 GEORGE A. OLAH ET AL 'Synthetic methods and reactions;82.Cyanuric chloride,a mild dehydrating agent in the preparation of nitriles from amides'

## Beschreibung

Die vorliegende Erfindung betrifft ein Verfahren zur Herstellung von 5-Cyano-4-(C₁₋₆-alkyl)-oxazolen. Diese Oxazole bilden eine wichtige Stoffgruppe. So ist beispielsweise 5-Cyano-4-methyl-oxazol ein wertvolles Zwischenprodukt bei der Synthese von Pyridoxin ( Vitamin B₆).

Es wurden bereits einige Verfahren zur Herstellung von 5-Cyano-4-methyl-oxazol durch Dehydratisierung von 5-Carbamoyl-4-methyl-oxazol beschrieben. So gelingt diese Dehydratisierung beispielsweise in Gegenwart von Phosphorpentoxid. Der Nachteil dieses Verfahrens ist jedoch die geringe Produktausbeute, was auf die bei dieser Reaktion sehr leicht auftretende Verkohlung zurückzuführen ist.

Eine Verbesserung dieses Verfahrens gelingt durch die Umsetzung von 5-Carbamoyl-4-methyl-oxazol mit Phosphorpentoxid in Gegenwart von Chinolin als Lösungsmittel [ US 3 222 374 ]. Auch dieses Verfahren beinhaltet Nachteile, die sich aus der Giftigkeit des Chinolins, seinem unangenehmen Geruch sowie seiner thermischen Instabilität ergeben. Zudem ist Chinolin ein relativ teures Lösungsmittel. Ein Problem stellen weiterhin die Regenerierung des Chinolins, die Verwendung von stöchiometrischen Mengen Phosphorpentoxid, die Aufarbeitung der Folgeprodukte des benötigten Phosphorpentoxids sowie deren umweltgerechte Entsorgung dar.

Ein weiteres bekanntes Verfahren zur Herstellung von 5-Cyano-4-methyl-oxazol besteht darin, das 5-Carbamoyl-4-methyl-oxazol mit einem Nieder-alkancarbonsäureanhydrid umzusetzen und das Reaktionsgemisch oder das aus diesem isolierte 4-Methyl-5-(N-niederalkanoyl-carbonyl)-oxazol einer Pyrolyse zu unterwerfen [ EP 0 010 697 ]. Die pyrolytische Endstufe hat jedoch gewisse Nachteile, insbesondere treten Korrosionsprobleme mit den Reaktorwerkstoffen auf und es bilden sich schwer rezyklisierbare Nebenprodukte.

Ein weiteres Verfahren [ US 4 026 902 ] besteht darin, dass 5-Carbamoyl-4-methyl-oxazol katalytisch unter Erhitzen in Anwesenheit von Phosphorpentoxid auf einem festen Träger zu 5-Cyano-4-methyl-oxazol dehydratisiert wird. Nachteilig bei diesem Verfahren sind die Handhabung von 5-Carbamoyl-4-methyl-oxazol, insbesondere die im Vordergrund stehende Sublimation und damit die Feststoffdosierung des schwerflüchtigen Ausgangsmaterials.

In der amerikanischen Patentschrift US 4 772 718 wird weiterhin die einstufige Ueberführung von 5-Carbonsäureethylester-4-methyl-oxazol in 5-Cyano-4-methyl-oxazol beschrieben. Bei diesem Verfahren wird der entsprechende Oxazolester in Gegenwart von Ammoniak und einem Zirkonoxid- oder Hafniumoxidkatalysator in der Gasphase zu 5-Cyano-4-methyl-oxazol umgesetzt. Nachteilig ist hier jedoch die Verwendung eines relativ teuren Katalysators sowie - zwecks Erreichen einer optimalen Reaktionsführung- das Einhalten sehr enger Reaktionbedingungen.

### Diskussion zum Stand der Technik:

George A. Olah et al. offenbaren in Synthesis Nr. 8, 657 - 658 (1980) u.a. die Dehydratisierung von Amiden und Thioamiden unter Verwendung von Cyanurchlorid und Dimethylformamid. Bei den zu dehydratisierenden Amiden handelt es sich um Alkyl-, Aralkyl- und Arylamide; heterocyclische Amide sind nicht vorgesehen.

Die schliesslich in der europäischen Patentanmeldung EP - 0 492 233 beschriebene Gasphasendehydratisation von 5-Carbamoyl-4-methyl-oxazol zu 5-Cyano-4-methyl-oxazol besitzt den Nachteil einer Reaktionstemperatur von 400°C - 500°C und muss bei einem Druck von 50 bis ca. 300 kPa durchgeführt werden.

Der vorliegenden Erfindung liegt nun die Aufgabe zugrunde, ein Verfahren zur Herstellung von 5-Cyano-4-(C₁₋₆-alkyl)-oxazolen bereitzustellen, das die Nachteile der vorbekannten Arbeitsweisen nicht aufweist, und gemäss welchem 5-Cyanol-4-(C₁₋₆-alkyl)-oxazol in kurzer Reaktionszeit unter milden Reaktionbedingungen in hoher Ausbeute erhalten wird.

Das erfindungsgemässe Verfahren zur Lösung dieser Aufgabe ist dadurch gekennzeichnet, dass man die Dehydratisierung von 5-Carbamoyl-4-(C₁₋₆-alkyl)-oxazol mit Cyanurchlorid und N,N-disubstituierten Formamiden, gegebenenfalls in Gegenwart eines weiteren Lösungsmittels, durchführt.

Der Ausdruck "(C₁₋₆-Alkyl" bedeutet, im Rahmen der vorliegenden Erfindung, gradkettige oder verzweigte Alkylreste mit 1 bis 6 Kohlenstoffatomen, wie z.B. Methyl, Ethyl, Propyl, iso-Propyl, Butyl, iso-Butyl, tert.Butyl, Pentyl, Hexyl und dergleichen. Geradkettige Alkylreste, wie insbesondere Methyl und Ethyl, sind bevorzugt.

N,N-disubstituierte Formamide können, im Rahmen der vorliegenden Erfindung, offenkettige Formamide wie N,N-Dimethylformamid, N,N-Diethylformamid, N,N-Di-n-propylformamid und dergleichen sein, sowie Formamide, bei denen die Substituenten Teil eines Ringes sind, wie z.B. N-Formylmorpholin, N-Formylpyrrolidin, N-Formylpiperidin und dergleichen. Bevorzugt ist N,N-Dimethylformamid.

Geeignete Lösungsmittel können, im Rahmen der vorliegenden Erfindung, Methyl-tert.-Butylether, Ethyl-tert.-Butylether, Tert.-Amylmethylether, N,N-Dimethylformamid, N,N-Diethylformamid, N,N-Di-n-propylformamid, N-Formylmorpholin, N-Formylpyrrolidin, N-Formylpiperidin und dergleichen sein. Bevorzugt ist Methyl-tert.-Butylether.

Die Dehydratisierung kann zweckmässigerweise in einem Temperaturbereich von etwa 0°C bis etwa 50°C, vorzugsweise bei etwa Raumtemperatur erfolgen.

Erfindungsgemäss kann 5-Carbamoyl-4-(C₁₋₆-alkyl)-oxazol in reinem N,N-disubstituiertem Formamid oder in einem der oben genannten Lösungsmittel, welches N,N-disubstituiertes Formamid enthält, suspendiert werden. Bevorzugt ist die Suspension in reinem N,N-Dimethylformamid. Die Umsetzung mit Cyanurchlorid in einem der oben genannten Lösungsmittel erfolgt bei einem 5-Carbamoyl-4-(C₁₋₆-alkyl)-oxazol : Cyanurchlorid-Mol-Verhältnis von etwa 3:1 bis 3:10, vorzugsweise bei einem 5-Carbamoyl-4-(C₁₋₆-alkyl)-oxazol : Cyanurchlorid-Mol-Verhältnis von etwa 3:1 bis 1:1, besonders bevorzugt bei einem 5-Carbamoyl-4-(C₁₋₆-alkyl)-oxazol : Cyanurchlorid-Mol-Verhältnis von etwa 2:1. Die Reihenfolge, in welcher die Reaktanden eingesetzt werden, ist nicht kritisch. Zweckmässig ist die oben beschriebene Abfolge.

Die Aufarbeitung des Rohprodukts an 5-Cyano-4-(C₁₋₆-alkyl)-oxazol kann nach Neutralisation der Reaktionsmischung z.B. durch Extraktion mit Lösungsmitteln wie Methyl-tert.-Butylether, Diethylether, Diisopropylether, Tert.-Amylmethylether, Ethylacetat, Methylacetat, Isobutylmethylketon, Pentan, Heptan, Dichlormethan und dergleichen erfolgen. Die Extraktion mit Methyl-tert.-Butylether ist dabei besonders bevorzugt. Zur Neutralisation eignen sich anorganische Basen wie NaOH, Na₂CO₃ und dergleichen, sowie organische Basen wie z.B. Amine. Geeignete Amine sind Ethylamin, Diethylamin, Triethylamin, Pyridin und dergleichen.

Mittels des erfindungsgemässen Verfahrens kann eine nahezu quantitative Umsetzung von 5-Carbamoyl-4-(C₁₋₆-alkyl)-oxazol zu 5-Cyano-4-(C₁₋₆-alkyl)-oxazol erzielt werden.

Das folgende Beispiel zur Herstellung von 5-Cyano-4-methyl-oxazol zeigt eine besonders vorteilhafte Ausführungsform des erfindungsgemässen Verfahrens auf.

### Beispiel:

22.88 g (198 mMol) 5-Carbamoyl-4-methyl-oxazol werden in 100 ml N,N-Dimethylformamid bei Raumtemperatur suspendiert und innerhalb von 15 Minuten mit 18.31 g (99.3 mMol) Cyanurchiorid in 250 ml Methyl-tert.-Butylether versetzt. Das Gemisch wird eine Stunde bei Raumtemperatur gerührt, wobei die zunächst gelbe Suspension nach orange übergeht. Anschliessend wird mit 50 ml gesättigter wässriger Na₂CO₃ - Lösung neutralisiert und die Phasen getrennt. Die wässrige Phase wird zweimal mit je 150 ml Methyl-tert.-Butylether extrahiert und die vereinigten organischen Phasen mit 250 ml dest. Wasser gewaschen, über Na₂SO₄ getrocknet, filtriert und am Rotationsverdampfer eingeengt. Es verbleibt ein oranges, flüssiges Rohprodukt. Die Ausbeute an 5-Cyano-4-methyl-oxazol beträgt 99.4% der Theorie. ( gaschromatographisch bestimmt)

## Patentansprüche

1. Verfahren zur Herstellung von 5-Cyano-(C₁₋₆-alkyl)-oxazolen durch Dehydratisierung von 5-Carbamoyl-4-(C₁₋₆-alkyl)-oxazolen, dadurch gekennzeichnet, dass man die Dehydratisierung mit Cyanurchlorid und N,N-disubstituierten Formamiden, gegebenenfalls in Gegenwart eines weiteren Lösungsmittels, durchführt.

2. Verfahren gemäss Anspruch 1, dadurch gekennzeichnet, dass man als N,N-disubstituiertes Formamid N,N-Dimethylformamid verwendet.

3. Verfahren gemäss Anspruch 1 oder 2, dadurch gekennzeichnet, dass die Dehydratisierung bei Temperaturen von etwa 0°-50°C, vorzugsweise bei etwa Raumtemperatur erfolgt.

4. Verfahren gemäss einem der Ansprüche 1 bis 3, dadurch gekennzeichnet, dass die Dehydratisierung bei einem 5-Carbamoyl-4-(C₁₋₆-alkyl)-oxazol : Cyanurchlorid-Mol-Verhältnis von etwa 3:1 bis 3:10, vorzugsweise von etwa 3:1 bis 1:1, besonders bevorzugt etwa 2:1 erfolgt.

5. Verfahren gemäss einem der Ansprüche 1 bis 4, dadurch gekennzeichnet, dass man die Dehydratisierung mit Cyanurchlorid und N,N-disubstituierten Formamiden in Gegenwart von Methyl-tert.-Butylether als weiteres Lösungsmittel durchführt.

## Claims

1. A process for the manufacture of 5-cyano-4-(C₁₋₆-alkyl)-oxazoles by dehydrating a 5-carbamoyl-4-(C₁₋₆-alkyl)-oxazole, characterized in that the dehydration is carried out with cyanogen chloride and N,N-disubstituted formamides, optionally in the presence of an additional solvent.

2. A process according to claim 1, characterized in that N,N-dimethylformamide is used as the N,N-disubstituted formamide.

3. A process according to claim 1 or 2, characterized in that the dehydration is carried out temperatures of about 0°-50°C, preferably at about room temperature.

4. A process according to any one of claims 1 to 3, characterized in that the dehydration is carried out using a 5-carbamoyl-4-(C₁₋₆-alkyl)-oxazole:cyanogen chloride molar ratio of about 3:1 to 3:10, preferably of about 3:1 to 1:1, particularly about 2:1.

5. A process according to any one of claims 1 to 4, characterized in that the dehydration with cyanogen chloride and N,N-disubstituted formamides is carried out in the presence of methyl tert.-butyl ether as the additional solvent.

## Revendications

1. Procédé de préparation des 5-cyano-4-(alkyle en C₁-C₆)-oxazoles par déshydratation des 5-carbamoyl-4-(alkyle en C₁-C₆)oxazoles, caractérisé en ce que l'on procède à la déshydratation à l'aide du chlorure de cyanuryle et de formamides N,N-disubstitués, éventuellement en présence d'un autre solvant.

2. Procédé selon la revendication 1, caractérisé en ce que le formamide N,N-disubstitué qu'on utilise est le N,N-diméthylformamide.

3. Procédé selon la revendication 1 ou 2, caractérisé en ce que la déshydratation est réalisée à des températures de 0 à 50°C environ, de préférence au voisinage de la température ambiante.

4. Procédé selon l'une des revendications 1 à 3, caractérisé en ce que la déshydratation est réalisée à un rapport molaire 5-carbamoyl-4-(alkyle en C₁-C₆)-oxazole:chlorure de cyanuryle d'environ 3:1 à 3:10, de préférence d'environ 3:1 à 1:1 et plus spécialement d'environ 2:1.

5. Procédé selon l'une des revendications 1 à 4, caractérisé en ce que l'on procède à la déshydratation à l'aide du chlorure de cyanuryle et de formamides N,N-disubstitués en présence d'éther de méthyle et de tert-butyle constituant l'autre solvant.
